# EUROPEAN PATENT SPECIFICATION

(11) **EP 2 519 211 B1**
(45) Date of publication and mention of the grant of the patent: **06.08.2014**
(21) Application number: 10818091.0
(22) Date of filing: 27.12.2010
(51) Int. Cl.: A61F 15/00

(54) **A THREE DIMENSIONAL CAGE ADAPTIVE DAMPENING DEVICE FOR PROTECTION OF EXTERNAL WOUNDS OF ANY TYPE AND DIMENSION.**
DREIDIMENSIONALE ANPASSUNGSFÄHIGE UND ABFEDERNDE EINHAUSUNG FÜR WUNDEN
DISPOSITIF D'AMORTISSEMENT EN FORME DE CAGE TRIDIMENSIONNELLE POUR LA PROTECTION DE PLAIES EXTERNES DE TOUT TYPE ET DE TOUTES DIMENSIONS

(30) Priority: 31.12.2009 IT PO20090015
(43) Date of publication of application: 07.11.2012
(73) Proprietor: Sellarulo, Andrea, 59016 Poggio a Caiano - Prato (IT)
(72) Inventor: Sellarulo, Andrea, 59016 Poggio a Caiano - Prato (IT)
(74) Representative: Fanfani, Stefano
(86) International application number: PCT/IB2010/003353
(87) International publication number: WO 2011/080572

(56) References cited:
- WO-A2-2006/130026
- CH-A- 269 938
- DE-A1- 2 535 760
- DE-A1- 10 138 783
- DE-U1-202007 015 548

## Description

### Field of the Invention

Object of the present invention is a three dimensional dampening adaptive cage to protect any type or size of external wounds, being used or not with waterproof plasters or various bandages of any type or dimension as indicated in the main claim, followingly being mentioned as "cage or three dimensional cage".

In particular, the structure of the cage presents caracteristics able to protect, without touching any external wounds, naturally, including those wounds of all joints, articulations and backs parts which are very difficult to heal, all thanks to the shock absorbing system which at the same time is an easy fitting and protection.

It is also possible to inject gaseous or liquid medical solutions into the cage in case of large wounds thanks to the use of a valvula.

### Background art

Problems are known about wounds of various type and dimensions, where contact between wounds and bandages have a long healing process, being known that in open air moderatly large wounds heal quicker.

### Aim of the Invention

The aim of the invention is to provide a dampening adaptive cover able to absorb shocks, to fit and to protect wounds, without having in anyway contact with external wounds of any sort, including articulation, joints, back and all areas difficult to treat, to guarantee a natural and quick recovery.

### Summay of the invention

This invention is expressed and characterized in the enclosed claims.

A first advantage is that in case of large wounds using the cage structure of the invention, even though its not enough avoiding contact with the wounds to heal quicker, we have an antiseptic,transpiring, protective and waterproof covering.

Furthermore, in case of large wounds thanks to a valvula device opening in the center of the "X" structure, by injecting gaseous or liquid medical solutions we can obtain less changes of bandages or even any with quicker healing times.

A further advantage is that the cage is easy to be manufactured and it does not require a specific construction technology to be made and to be set in function, it being realised in medical silicon or medical plastic, or any other material that is compatible to skin contact.

In a preferred embodiment it is also possible to dress the upper part of the cage with poliurethane and to cover the underside with a suitable adhesive.

In the described embodiment, the cage is made in one whole piece, in a mould, shaped as a half ellipse three dimensional, with the lower parts, seen by the underside, shaped as two squared brackets, joined by a lightly curved staff.

From the four extreme angles of the square brackets it departs a sole flexible crossing bridge structure shaped as an "X", having in the center a valvula device being used to receive medical solutions inside the cage.

Between the upper part and the base of squared brackets we have a space shaped as a quarter ellipse three dimensional to create a sort of shock absorber which, when it receives a pressure or expansion, it shows a lightly stretching and lowering of the structure so that when the cage arrives to its end stroke point it does not still permit any contact between the cage and the wound. In this way the common situation in which the wound looks "boiled" is avoided.

The approaching between the upper central "X" portion and the lightly curved staff of the base, creates adaptability of the cage because the cage is made in flexible material adapting itself into the chosen positions such as on articulations, joints, shoulders and so on, offering a protection against any contacts for any type or size of external wounds, but also from any possible detachment of plasters or bandages which often, in various parts of the body such articulation joints and any other difficult areas, may loose adherence when the possibility to adapt to any movements of the personal injury is not provided.

### List of drawings

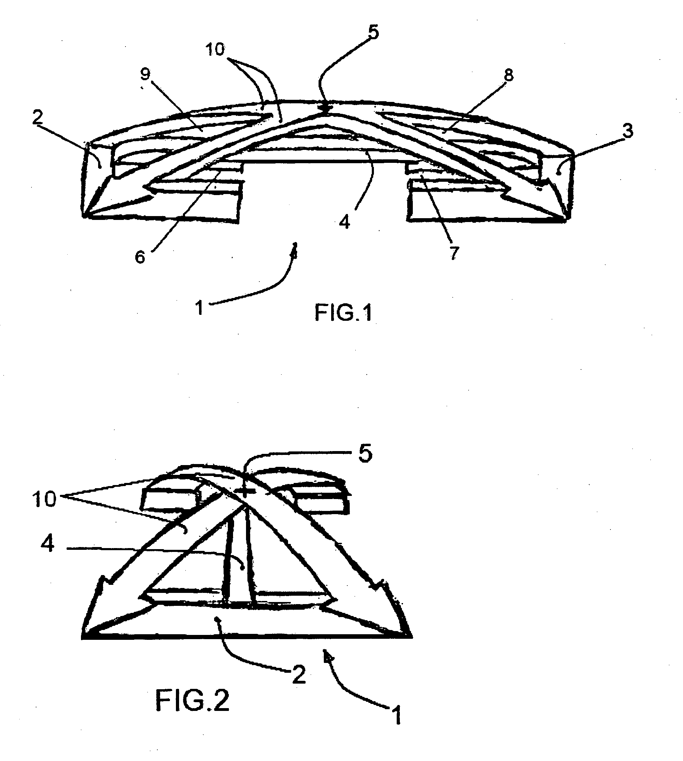

- figures 1, 2 show a prospective view of three dimensional cage;

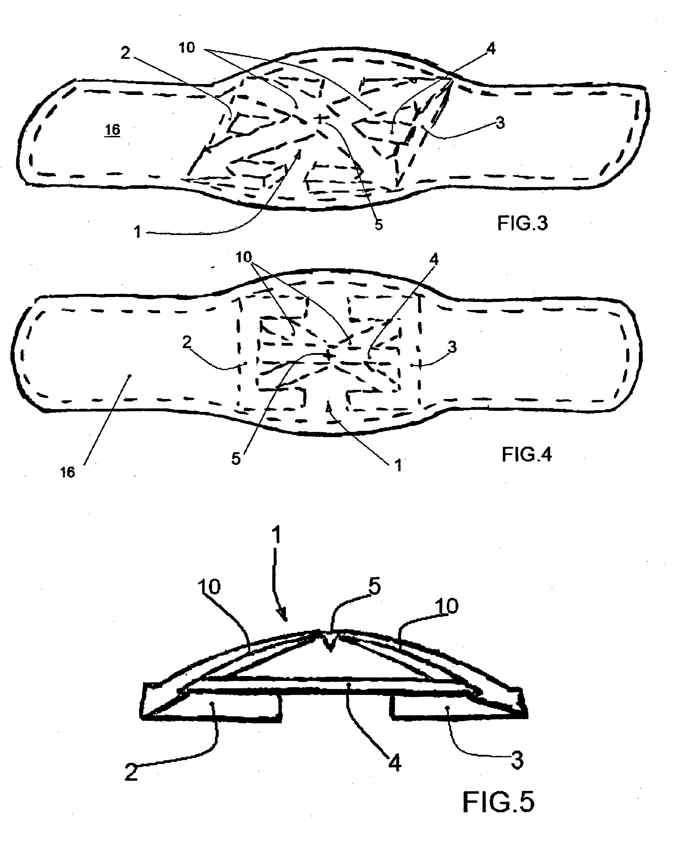

- figures 3, 4 respectively show an upper view and a lower view of a possible application of the cage;
- figure 5 shows a right side view of the cage of fig.2.

### Detailed description of the invention

With reference to the enclosed drawings, the cage 1 of the invention is a one piece moulded structure shaped as an half threedimensional ellipse having lower portions 2, 3, shaped as squared braquets and joined by a slightly curved staff 4.

From the four extreme angles of the square bracket it departs a one and only flexible bridge structure 10 shaped like an "X" having in the center a valvula device 5.

Between the base of squared brackets 2, 3 and the bridge structure 10 a three dimension open space 6, 7, 8, 9 is provided shaped as a quarter ellipse to create a dampening effect, so that at the moment of receiving a pressure or extension the cage exhibits a light stretching and lowering movement which, when the structure arrives to the end of its stroke, does not permit any contact between the three dimensional cage and the wound, protecting the latter.

Advantageously, the one piece flexible upper bridge structure 10 crossed as an "X" and the lightly curved staff 4 in the lower part of the cage, are made by flexible materials and they adapt themselves into the chosen positions, such as on articulation joints, shoulders and others, protecting any external wounds of any dimensions, ad avoiding the detachment of the cage from the skin.

Preferably, the cage could be made of medical silicon, medical plastic or any other material compatible for skin contact, both flexible or rigid, depending on the area to be protected.

The upper part 10 may be coated by poliurethane or by other material, and the base may be coated by any suitable adhesive covering.

In the fig.s 3, 4 an upper and a lower view of a possible application of the cage are respectively shown.

In the drawings, the cage 1 has been put inside in the center of the under part of a plaster or bandage, forming in this case an elliptic shape, but it can also be used in various shapes. The cage can also be used separatly without plasters or bandages being clear that the upper part must be dressed in material like poliurethane and the base with a suitable adhesive.

## Claims

1. A three dimensional cage adaptive dampening device for protection of external wounds of any type and dimension, made in one only moulded piece, and shaped as a three dimensional adaptive dampening half ellipse, **characterized by** four dampening parts and two parts shaped as squared brackets (2, 3) jointed in the middle by a lightly curved staff (4), in which from the four extremity corners of the square bracket parts it departs one and only structure shaped as a flexible bridge crossed as an "X" (10) having in the center a valvula device to receive pharmaceutical drugs (5)."

2. The device according to one of the preceding claims, in which between the base of the square brackets and the structure of the bridge, an open space is provided shaped as a three dimensional quarter ellipse (6, 7, 8, 9), creating a dampening, that at the moment of pressure or extension exhibits a light stretching movement that lowers the structure which arrives to end of its stroke getting no contact betweeen the three dimensional cage and the wound, protecting it.

3. The device according to one of the preceding claims, in which the one piece flexible bridge structure crossing as an "X" in the upper part, and the lightly curved staff in the lower part of the cage, they being made with flexible materials adapt themselves into assigned positions, such as articulation joints, shoulders and others, protecting any external wounds of any dimensions, avoiding the cage detachment from the skin.

4. The device according to one of the preceding claims, in which the material to be used could be medical silicon, medical plastic or any other material compatible at skin contact, flexible or rigid, depending on the area to be protected.

5. The device according to one of the preceding claims, in which the upper part maybe coated by poliuretane or other material and adhesive covering, suitable for the base.

## Patentansprüche

1. Dreidimensionale anpassungsfähige und abfedernde Einhausung für den Schutz von äußerlichen Wunden jeglicher Art und Dimension, ausgebildet in einem einzigen geformten Stück und ausgestaltet als dreidimensionale anpassungsfähige und abfedernde Halbellipse, **gekennzeichnet durch** vier abfedernde Teile und zwei Teile, die als rechtwinklige Bügel (2, 3) ausgestaltet sind, verbunden in der Mitte **durch** einen leicht gekrümmten Stab (4), wobei von den vier äußersten Ecken der rechtwinkligen Bügelteile eine einzige Konstruktion beginnt, ausgestaltet als flexible, wie ein "X" gekreuzte Brücke (10), aufweisend in der Mitte eine Klappenvorrichtung, um Arzneimittel (5) aufzunehmen.

2. Einhausung nach einem der vorhergehenden Ansprüche, wobei zwischen der Basis der rechtwinkligen Bügel und der Konstruktion der Brücke ein offener Bereich bereitgestellt ist, ausgestaltet als dreidimensionale Viertelellipse (6, 7, 8, 9), schaffend eine Abfederung, die bei einem Druck- oder Ausdehnungsmoment eine leichte Streckbewegung aufweist, die die Konstruktion senkt, die an das Ende ihres Hubs gelangt und nicht mit der dreidimensionalen Einhausung und der Wunde in Kontakt gelangt und diese schützt.

3. Einhausung nach einem der vorhergehenden Ansprüche, wobei die aus einem Stück bestehende flexible Brückenkonstruktion, die wie ein "X" im oberen Teil gekreuzt ist, und der leicht gekrümmte Stab im unteren Teil der Einhausung aus flexiblen Materialien bestehen, die sich selbst in zugewiesene Positionen einpassen wie Gelenkverbindungen, Schultern und Sonstiges und alle äußerlichen Wunden jeglicher Dimension schützen und vermeiden, dass die Einhausung von der Haut abgelöst wird.

4. Einhausung nach einem der vorhergehenden Ansprüche, wobei das einzusetzende Material medizinisches Silikon, medizinischer Kunststoff oder irgendein anderes Material sein kann, das bei Hautkontakt kompatibel ist, flexibel oder steif, je nach dem zu schützenden Bereich.

5. Einhausung nach einem der vorhergehenden Ansprüche, wobei der obere Teil mit Polyurethan oder einem anderen Material und einer haftenden Abdeckung, geeignet für die Basis, beschichtet sein kann.

## Revendications

1. Un dispositif amorti adaptif tridimensionnel pour la protection de blessures extérieures de n'importe quel type et dimensions, réalisé en une pièce unique estampée, ayant la forme d'une demi-ellipse amortie adaptive tridimensionnelle, **caractérisé par** quatre parties amorties et deux parties ayant la forme d'une parenthèse carrée (2, 3) jointes an milieu par une tige légèrement cambrée (4), dans lequel des quatre coins extrêmes se départit une seule structure flexible ayant la forme d'un pont croisé à "X" (10) dans le centre de laquelle il y a un dispositif à soupape capable de recevoir des préparations pharmaceutiques (5).

2. Un dispositif selon une des revendications précédentes, dans lequel entre la base des parenthèses carrées et la structure du pont il y a un espace libre à forme d'un quart d'ellipse tridimensionnelle (6, 7, 8, 9), tel à créer une sorte d'amortisseur lequel, tout en recevant de la pression ou un allongement, subit un léger mouvement de détente qui fait baisser la structure laquelle, en arrivant à sa fin de course, évité le contact entre la cage tridimensionnelle et la blessure, de façon à protéger celle-ci.

3. Un dispositif selon une des revendications précédentes, dans lequel la structure à pont flexible monopièce croisée à "X" supérieure et la tige légèrement cambrée dans la section inférieure de la cage, étant réalisées par des matériaux souples, s'adaptent aux positions à elles assignées, par exemple jointures articulaires, épaules, et ainsi de suite, de façon à protéger toutes blessures extérieures de n'importe quelles dimensions, tout en évitant que la cage se détache de la peau.

4. Un dispositif selon une des revendications précédentes, dans lequel le matériel à utiliser pourrait être de la silicone médicale, de la plastique médicale, ou tout autre matériel compatible pour le contact avec la peau, soit souple soit rigide, en fonction de l'aire à protéger.

5. Un dispositif selon une des revendications précédentes, dans lequel la partie supérieure peut être revêtue de polyuréthane ou d'un autre matériel et revêtement adhésif approprié pour la base.
